Europäisches Patentamt

European Patent Office

Office européen des brevets

(19)

(11) Veröffentlichungsnummer: **0 131 877**
**A2**

(12) ## EUROPÄISCHE PATENTANMELDUNG

(21) Anmeldenummer: 84107957.7

(22) Anmeldetag: 06.07.84

(51) Int. Cl.⁴: **G 01 N 21/53**
**G 08 B 17/10**

(30) Priorität: 15.07.83 CH 3900/83
03.10.83 CH 5367/83

(43) Veröffentlichungstag der Anmeldung:
23.01.85 Patentblatt 85/4

(84) Benannte Vertragsstaaten:
BE CH DE FR GB IT LI NL

(71) Anmelder: CERBERUS AG
Alte Landstrasse 411
CH-8708 Männedorf(CH)

(72) Erfinder: Troup, Alan
Herweg 30
CH-8708 Männedorf(CH)

(72) Erfinder: Strässler, Sigfrid
Segelhalde 40
CH-5405 Dättwil-Baden(CH)

(72) Erfinder: Güttinger, Hannes
Obere Lattenbergstr. 20d
CH-8712 Stäfa(CH)

(72) Erfinder: Pfister, Gustav
Forbüelstr. 17
CH-8707 Uetikon a/See(CH)

(74) Vertreter: Tiemann, Ulrich, Dr.-Ing. et al,
c/o Cerberus AG Patentabteilung Alte Landstrasse 411
CH-8708 Männedorf(CH)

(54) **Anordnung zur Detektion einer in einem Gas enthaltenen Fremdkomponente, sowie deren Verwendung.**

(57) Zur Detektion einer Fremdkomponente in einem Gas, z.B. von Brandaerosol oder Rauch in Luft, ist ein Aerosoldetektor, z.B. eine Transmissionsmessstrecke (17) in einer Messkammer (7) vorgesehen, die mit einer Referenzkammer (5) mit aerosolfreiem Referenzgas in Verbindung steht. Durch eine periodische Volumenänderung der Referenzkammer (5) wird das Gas in der Messkammer (7) periodisch gegen sauberes Gas aus der Referenzkammer (5) ausgetauscht, so dass bei Anwesenheit von Aerosol die Aerosoldichte in der Messkammer (7) und das Ausgangssignal des Aerosoldetektors moduliert werden. Die Modulation, d.h. der Wechselanteil des Ausgangssignales ist ein Mass für die Aerosolkonzentration oder die Rauchdichte.

Eine besonders geeignete Verwendung ist die Rauchdetektion in Luft zum Zwecke der Brandmeldung oder die Kontrolle der Luftverschmutzung.

FIG. 1

CERBERUS AG                    CH-8708 Männedorf, Schweiz

Anordnung zur Detektion einer in einem Gas enthaltenen
Fremdkomponente, sowie deren Verwendung

Die Erfindung betrifft eine Anordnung zur Detektion einer
in einem Gas enthaltenen Fremdkomponente mit einer für das
zu untersuchende Gas zugänglichen Messkammer, die einen in
Abhängigkeit vom Fremdkomponentengehalt ein Ausgangssignal
abgebenden Sensor aufweist, und einer das Ausgangssignal
auswertenden elektrischen Schaltung, und deren Verwendung.

Eine solche Anordnung lässt sich im Prinzip zur Detektion
beliebiger fester, flüssiger oder gasförmiger Fremdkomponenten in einem Gas verwenden, speziell von Schwebeteilchen
oder Aerosolen. Der Sensor ist dabei auf die Fremdkomponente
abgestimmt und kann z.B. als die Schwächung elektromagnetischer Strahlung ausnützende Transmissionsmessstrecke ausgebildet sein. Von besonderem Vorteil ist die Verwendung der
Anordnung zur Detektion von Brandaerosolen oder Rauchpartikeln in Luft, beispielsweise zum Zwecke der Brandmeldung.

Zur Brandaerosol- oder Rauch-Detektion wurden bisher vielfach
Streustrahlungs-Detektoren verwendet, bei denen die an Rauchpartikeln gestreute elektromagnetische Strahlung, d.h. sichtbares Licht oder infrarote Strahlung, von einem ausserhalb des
direkten Strahlenganges angeordneten Streustrahlungsempfänger
registriert wird. Ein solcher Detektor kann sehr empfindlich
eingerichtet werden, da bei Abwesenheit von Rauch keine Streustrahlung auftritt, das Signal also nahezu Null ist, und bei
Anwesenheit von Rauch daher nur eine kleine Abweichung vom
Nullwert festzustellen ist, was messtechnisch keine besonderen Schwierigkeiten bereitet. Nachteilig ist jedoch, dass derartige Rauchdetektoren nur auf streuenden Rauch, beispielsweise auf weissen, stark wasserdampfhaltigen Rauch reagieren, jedoch nicht oder kaum auf schwarzen, vorzugsweise strahlungsabsorbierenden, jedoch nicht oder nur wenig streuenden Rauch.

Zur Vermeidung dieses Nachteiles und zur gleichzeitigen Detektion von strahlungsstreuendem und von strahlungsabsorbierendem Rauch hat es sich als vorteilhaft erwiesen, die Transmissionsänderung eines Gases, bzw. der Luft, als Kriterium für die Anwesenheit von Aerosolen zu verwenden. Hierbei wird die Strahlungsschwächung der von einer Strahlungsquelle ausgesandten und nach Durchquerung einer Transmissionsmessstrecke von einem Strahlungsempfänger aufgenommenen Strahlung zur Rauchdetektion ausgewertet. Da hierzu aber eine relativ kleine Abweichung von einem grossen Normalwert zu bestimmen ist, ist zur zuverlässigen Detektion einer kleinen Rauchkonzentration eine relativ grosse Länge der Transmissionsmessstrecke erforderlich, in der Praxis im Meter-Bereich, was die praktische Verwendung erheblich behindert, und ohne weitere Vorkehrungen nahezu verunmöglicht.

Zur Beseitigung dieses Nachteiles und zur Vermeidung derart grosser Abmessungen ist es bekannt, einen gefalteten Strahlengang zu verwenden, bei dem die Strahlung durch Reflektoren oder Spiegel umgelenkt wird, sodass mit einer genügenden Anzahl von Reflektoren die Abmessungen des Rauchdetektors auf einen praktisch tragbaren Wert in der Grössenordnung von etwa 10 cm reduziert werden konnten. Hierbei tritt jedoch das Problem auf, dass durch eine Verminderung der Reflexionsfähigkeit der Spiegel, beispielsweise infolge Verstaubung der Oberflächen, ebenso wie durch eine Alterung der Strahlungsquelle, eine Strahlungsschwächung durch Rauch vorgetäuscht und ein fehlerhafter Alarm ausgelöst wird.

Der in DE-A 31 17 757 beschriebene Rauchdetektor versucht
diesen Nachteil der langsamen Verstaubung der Optik durch
Verwendung einer besonderen Stabilisierungs- und Nachführungs-Elektronik zu vermeiden, so dass langsame Aenderungen
kompensiert werden, und nur bei schnellen Aenderungen eine
Signalgabe erfolgt. Dabei ist jedoch ein erheblicher Schaltungsaufwand erforderlich, und zudem kann eine langsame
Rauchentwicklung nicht von einer Verstaubung unterschieden
und daher auch nicht erkannt und detektiert werden.

Stattdessen wurden verschiedentlich, z.B. in DE 1 038 454
oder CH 561 942, Rauchdetektoren vorgeschlagen, die neben
der eigentlichen Transmissionsmessstrecke eine Referenzmessstrecke mit unterschiedlicher Strahlungsschwächung oder
verschiedener optischer Weglänge aufweisen. Da hierbei aber
eine sehr kleine Differenz zweier grosser Signale gebildet
werden muss, ist ein erheblicher Aufwand für die elektrische
Stabilisierung erforderlich. Ausserdem ist eine genaue
mechanische Justierung und deren Aufrechterhaltung während
längerer Zeiten notwendig, da solche Rauchdetektoren zur
mechanischen Instabilität und zur Erschütterungsempfindlichkeit neigen.

Bei der in DE-A 1 942 942 beschriebenen Rauchdichte-Messeinrichtung wird zur Vermeidung der Nachteile eines Zweistrahlverfahrens auf einen Referenzstrahlengang verzichtet
und stattdessen die Weglänge der Transmissionsmessstrecke
mechanisch moduliert, indem z.B. eine transparente Abschluss-
scheibe der Transmissionsmesskammer oder ein anderes Bauteil,
wie die Strahlungsquelle, der Strahlungsempfänger, oder ein
Reflektor, in eine Schwingung mit bestimmter Amplitude versetzt wird, so dass die optische Weglänge periodisch zwischen
zwei Werten schwankt. Der Wechselanteil des Empfänger-Ausgangssignales ist dabei ein Mass für die Rauchdichte.

- 4 -

0131877

Die Längenmodulation umfasst hierbei jedoch nur einen kleinen Teil der Gesamtlänge der Transmissionsmessstrecke. In der Praxis, z.B. bei der Anwendung zur Brandmeldung, sind jedoch zur Erzielung einer genügenden Rauch-Empfindlichkeit recht grosse Modulations-Amplituden wenigstens im Zentimeter-Bereich notwendig. Die Zuverlässigkeit und die Energie-Aufnahme eines Schwingungsgeneratorsystems mit so grosser Amplitude ist jedoch mit den an Rauchdetektoren mit langzeitigem, möglichst wartungsarmen und energiesparenden Betrieb gestellten Anforderungen nicht vereinbar.

Einen anderen Weg geht die in US 2 486 622 offenbarte Rauchdetektionsanordnung, bei der mittels eines Ventilators über ein steuerbares Ventil das zu untersuchende Gas abwechselnd direkt und über ein rauchabsorbierendes Filter durch eine Messkammer hindurch transportiert wird. Der Wechselanteil des Ausgangssignals des als Transmissionsmessstrecke oder Streulichtdetektor ausgebildeten Sensors ist dabei ein Mass für die Rauchkonzentration. Nachteilig ist hierbei jedoch, dass der Ventilator-Motor dauernd in Betrieb sein und ständig eine beträchtliche Luftmenge fördern muss. Ein langzeitig funktionssicherer, wartungsarmer und energiesparender Betrieb ist daher ebenfalls nicht gewährleistet. Hinzu kommt, dass das erforderliche Filter nur eine bestimmte Menge von Aerosol, Rauch oder Staubpartikel zu absorbieren vermag. Ständig in der Luft anwesende schwache Fremdpartikelkonzentrationen kumulieren sich daher rasch am Filter und machen dieses in kurzer Zeit unwirksam, so dass im Ernstfall, z.B. bei einem Brandausbruch, auftretender Rauch nicht detektiert wird, wenn das Filter nicht regelmässig in kurzen Zeitabständen ausgetauscht, gereinigt oder regeneriert wird.

Die Erfindung setzt sich die Aufgabe, die vorstehend erwähnten Nachteile des Standes der Technik zu beseitigen, und insbesondere eine Detektions-Anordnung der eingangs angegebenen Art zu schaffen, die kleinere Abmessungen aufweist, und bei der ohne komplizierte, aufwendige und störanfällige optische und mechanische Anordnungen und elektrische Komponenten und ohne genaue Justierung eine genaue, langzeitstabile und störsichere Detektion, insbesondere von Rauch erreicht wird.

C 269 a

Erfindungsgemäss wird diese Aufgabe dadurch gelöst, dass eine für das zu untersuchende Gas nicht direkt zugängliche Referenzkammer vorgesehen ist, die ein die zu detektierende Fremdkomponente nicht oder in geringerem Anteil aufweisendes Referenzgas enthält, die mit der Messkammer mit wenigstens einer Oeffnung in Verbindung steht, und die einen Luftbewegungsgenerator aufweist, der periodisch abwechselnd Referenzgas aus der Referenzkammer in die Messkammer drückt, wobei das Gas in der Messkammer zumindest teilweise durch Referenzgas ersetzt wird, und das Referenzgas wieder in die Referenzkammer zurückzieht, wobei wiederum das zu untersuchende Gas in die Messkammer eindringt, und dass die elektrische Schaltung den Wechselanteil des Ausgangssignales des Sensors als Kriterium für den Fremdkomponentengehalt auswertet.

Besonders günstig ist es, den Luftbewegungsgenerator so auszubilden, dass er das Volumen der Referenzkammer periodisch ändert und dabei periodisch Referenzgas, z.B. saubere Luft, aus der Referenzkammer in die Messkammer drückt und wieder zurücksaugt. Mit Vorteil kann dazu eine Referenzkammer-Wand als schwingende Membran ausgebildet sein. Bei einer relativ grossen schwingenden Fläche der Wand lässt sich so schon bei geringer Schwingungsamplitude und geringem Energiekonsum ein fast vollständiger oder zumindest weitgehender periodischer Gasaustausch in der Messkammer erreichen. Dabei ist es zweckmässig, wenn das Referenzkammer-Volumen gleich oder besonders vorteilhaft grösser ist als das Messkammer-Volumen.

Der Sensor kann dabei mit Vorteil als luftzugängliche Transmissionsmessstrecke ausgebildet sein. Ein besonders guter Gasaustausch wird hierbei erreicht, wenn die Messkammer eine langgestreckte Form besitzt und praktisch nur die Transmissionsmessstrecke umfasst, wobei die Oeffnungen zur Referenzkammer als parallel zur Transmissionsmessstrecke verlaufender Schlitz ausgebildet sind, wodurch ein besonders schneller Luftaustausch erreicht wird.

Die Erfindung, sowie zweckmässige Weiterbildungen derselben, wird an Hand der in den Figuren dargestellten Ausführungsbeispiele erläutert.

Figur 1 zeigt einen Rauchdetektor im Schnitt,

Figur 2A und B zeigen dafür geeignete Auswerteschaltungen,

Figur 3 zeigt ein Beispiel einer Referenzkammer,

Figur 4 zeigt ein weiteres Beispiel einer Referenzkammer,

Figur 5 zeigt ein Beispiel einer Messkammer,

Figur 6 zeigt ein weiteres Beispiel einer Messkammer.

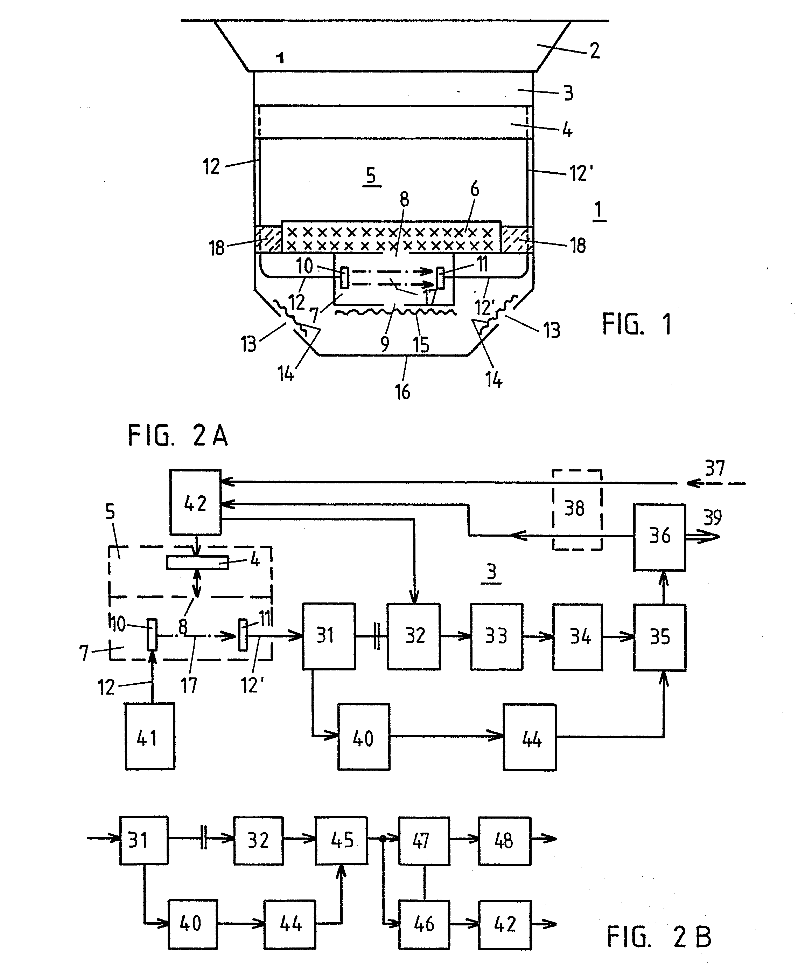

Die in Figur 1 dargestellte, speziell zur Rauchdetektion und zur Brandmeldung geeignete Anordnung zur Aerosoldetektion besteht aus einem Detektor-Einsatz 1, der in einen Sockel 2, z.B. einen üblichen Brandmelder-Sockel einsetzbar ist. Der Detektor-Einsatz 1 enthält eine elektrische Steuer- und Auswerteschaltung 3, oder zumindest einen Teil derselben, wobei der Rest der Schaltung in einer über Leitungen mit dem Sockel 2 verbundenen, nicht dargestellten Signalzentrale untergebracht sein kann. Von der Schaltung 3 wird ein Luftbewegungsgenerator 4 angesteuert, der eine Wand der anschliessenden Referenzkammer 5, oder einen Wandteil derselben bildet und deren Volumen periodisch mit einer bestimmten Frequenz, vorzugsweise zwischen 0,1 und 10 Hz, z.B. 2 Hz, ändert. Der Luftbewegungsgenerator 4 kann z.B. als elektromagnetisch, elektrostatisch, piezoelektrisch oder thermomechanisch erregbare Membran in der Art eines Lautsprechers ausgeführt sein, oder eine Piezo-Folie vom Typ PVDF aufweisen, oder ein bimorphes piezoelektrisches oder bimetallisches Element enthalten. In einem praktisch ausgeführten Beispiel erwies sich als Luftbewegungsgenerator ein Philips Woofer AD 4060/W4 als gut geeignet. Die Referenzkammer dient hierbei als Reservoir für ein Referenzgas, das das zu detektierende Aerosol nicht oder nur mit geringem Anteil enthält, beispielsweise saubere, nicht rauchhaltige Luft.

Die Referenzkammer 5 steht mit der eigentlichen Messkammer 7, die im dargestellten Beispiel als Transmissionsmesskammer ausgebildet ist, über eine oder mehrere Verbindungsöffnungen 8 geringen Querschnittes von ca. 0,1 cm$^2$ in Verbindung. Die Messkammer weist dabei ein kleineres Volumen von ca. 0,75cm$^3$ auf als die Referenzkammer mit ca. 100 cm$^3$, so dass das Volumen-Verhältnis mehr als 1 : 100 beträgt. Bei einer Schwingung des Luftbewegungsgenerators, bzw. der Referenzkammer-Wand 4 wird nun ein der Schwingungsamplitude entsprechendes Volumen von Referenzgas, d.h. reiner oder weniger rauchhaltiger Luft von der Referenzkammer 5 durch die Oeffnungen 8 in die Messkammer 7 hineingedrückt und verdrängt dort die zu untersuchende Luft periodisch.

In der Referenzkammer 5 ist vor den zur Messkammer 7 führenden Oeffnungen 8 ein luftdurchlässiges, jedoch rauchundurchlässiges Filter 6 vorgesehen, das z.B. aus einer Glasfibermatte bestehen kann, welche eine gasspezifische Oberfläche aufweist, an die sich die Rauchteilchen anlagern können. Das Filter 6 verhindert, dass bei der periodischen Hin- und Her-Bewegung der Luft von der Referenzkammer 5 in die Messkammer 7 Rauchaerosol in die Referenzkammer 5 mitgenommen wird, so dass stets reine Luft als Referenzgas zur Verfügung steht. Ausserdem kann in der Referenzkammer ein Rauchabsorptionsmittel 18 vorgesehen sein, das z.B. Aktivkohle, Glasfibern, Filtertuch, Filterpapier, elektostatische Filter oder rauchadsorbierende Oberflächen enthalten kann, und die doch noch in die Referenzkammer eingedrungenen Spuren von Rauch absorbiert. Bei Anordnung in der Nähe der Oeffnungen 8 kann dabei sogar das Filter 6 entfallen und die Oeffnungen offen bleiben.

Die Messkammer ist im dargestellten Beispiel als Transmissionsmesskammer ausgebildet und enthält eine Strahlungsquelle 10, die elektromagnetische Strahlung, wie sichtbares Licht oder infrarote Strahlung aussendet, z.B. vom Typ Siemens SFH 400 und einen Strahlungsempfänger 11, der die direkte Strahlung der Strahlungsquelle 10 aufnimmt, und z.B. vom Typ Siemens

BPY 64 P sein kann. Strahlungsquelle 10 und Strahlungsempfänger 11 sind mit elektrischen Leitungen 12 und 12' mit der elektrischen Schaltung 3 verbunden. Der Zwischenraum 17 zwischen der Strahlungsquelle und dem Strahlungsempfänger bildet die Transmissionsmessstrecke mit einer wirksamen Länge von nur wenigen cm. Es sei bemerkt, dass Strahlungsquelle und Strahlungsempfänger auch ausserhalb der Messkammer 7 angeordnet sein können und die Messkammer durch strahlungsdurchlässige Fenster durchstrahlt sein kann. Die Messkammer 7 ist im dargestellten Beispiel von einem Gehäuse 16 umgeben, mit dessen Innerem sie über weitere Oeffnungen 9, die mit einem Staubabscheider 15 versehen sein können, in Verbindung steht. Das Gehäuse 16 weist seinerseits Oeffnungen 13 auf, durch die die Aussenluft über weitere Staubabscheider 14 bzw. eine labyrinthartige Lichtfalle und windhemmende Abschirmung in das Innere eindringen kann.

Beim Betrieb der beschriebenen Anordnung wird periodisch mit einer bestimmten Frequenz, z.B. 2 Hz, Luft zwischen der Referenzkammer 5 und der Messkammer 7, und damit auch zwischen der Messkammer 7 und der Aussenatmosphäre hin und her bewegt. Dabei sollte zweckmässigerweise das totale bewegte Luftvolumen etwa dem Volumen der Messkammer 7 entsprechen. Damit wird das Messkammer-Volumen zumindest teilweise, im Idealfall möglichst vollständig periodisch ausgetauscht, und es befindet sich abwechselnd Aussenluft und saubere Luft aus der Referenzkammer 5 in der Messkammer 7, und damit auch in der Transmissionsmessstrecke 17 zwischen der Strahlungsquelle 10 und dem Strahlungsempfänger 11. Dabei hängt das bewegte Volumen von der speziellen geometrischen Ausbildung der beiden Kammern 5 und 7 und der Oeffnungen 8 und den Eigenschaften des Filters 6 ab.

Solange sich in der Aussenluft keine strahlungsschwächenden Rauchaerosole befinden, bleibt die Intensität der vom Strahlungsempfänger 11 empfangenen Strahlung unverändert und gleichförmig. Sobald aber die Aussenluft Rauch enthält, wechselt die Luft in der Messkammer 7 periodisch zwischen rauchfreier oder rauchärmerer Luft aus der Referenzkammer 5 und rauchhaltiger Luft aus der Aussenatmosphäre. Daher ändert sich nunmehr die Intensität der empfangenen Strahlung periodisch mit der Frequenz der Luftwechsel in der Messkammer 7, d.h. der elektrischen Auswerteschaltung 3 wird über Leitung 12' ein Wechselsignal mit der Betriebsfrequenz des Luftbewegungsgenerators 4 zugeführt. Diesem Signal ist bei dem bevorzugten Betrieb der Strahlungsquelle 10 mit Gleichstrom ein Gleichspannungssignal überlagert, bzw. bei dem auch möglichen Impulsbetrieb bei wesentlich verschiedener Frequenz ein Wechselsignal abweichender Frequenz. Das Wechselsignal bei der Luftbewegungsfrequenz kann von der Auswerteschaltung auf einfache Weise vom Gleichspannungsanteil oder vom Wechselsignal mit abweichender Frequenz getrennt werden und ist unmittelbar proportional zur Rauchdichte.

Für das beschriebene Verfahren ist es wesentlich, dass im Normalfall, d.h. bei rauchfreier Luft, das ausgewertete Signal etwa Null ist, und nur dann ein praktisch von Null verschiedenes Signal erzeugt wird, wenn Rauch in der Aussenluft vorhanden ist. Das Signal entsteht also nicht aus einer Differenzbildung zweier Signale, und dementsprechend sind die Anforderungen an eine Stabilisierung wesentlich geringer als bei vorbekannten Verfahren. Dabei ist es ausserordentlich vorteilhaft, dass sich mit einer sehr geringen Schwingungsamplitude des Luftbewegungsgenerators im Zehntelmillimeter-Bereich und entsprechend geringem Leistungsbedarf ein ausreichender Luftwechsel in der Messkammer und Transmissionsmessstrecke erreichen lässt. Die damit erzielte Modulationsamplitude der Strahlung und des Ausgangssignales erreicht dabei fast den maximal möglichen Wert und ist zumindest um eine Grössenordnung grösser als bei der vorbekannten direkten Längenmodulation der Transmissionsmessstrecke. Es zeigte sich bei einem praktisch ausgeführten Beispiel, dass sich auf die beschriebene Weise mit einer Transmissionsstrecken-Länge von weniger als 10 cm eine Rauchdichte von 0,4 %/m mit einer einfachen Auswerteschaltung ohne Aufwand sicher und störunanfällig detektieren lässt.

Für die Auswertung des Wechselsignales kann im Prinzip eine beliebige dem Fachmann bekannte Wechselsignal-Mess- oder Alarm-Schaltung verwendet werden. Fig. 2A zeigt ein geeignetes Beispiel in Blockform, wobei die Auswahl der handelsüblichen Komponenten dem Fachmann geläufig ist.

Bei dieser Schaltung wird von einer Gleichstromquelle 41 die Strahlungsquelle 10 über die Leitung 12 betrieben und sendet Strahlung über die Transmissionsstrecke 17 zum Strahlungsempfänger 11. Dessen Ausgangssignal gelangt über die Leitung 12' über einen Stromwandler 31 zu einem Bandpassverstärker 32, der vorzugsweise nur die Frequenz des Luftwechselgenerators 4, bzw. dessen Treibers 42 durchlässt, und dann über einen Gleichrichter 33, einen Integrator 34 zu einem Komparator 35, der das integrierte Signal mit einem Referenzsignal vergleicht und eine Alarmschaltung 36 ansteuert, sobald das integrierte Signal einen vorgegebenen Schwellenwert übersteigt, d.h. wenn die Rauchdichte während einer durch die Zeitkonstante des Integrators 34 bestimmten Zeit über einem bestimmten Wert bleibt. Zusätzlich kann vom Strahlungsempfänger ein Drift-Kompensator 40 angesteuert werden, der die Referenzspannungsquelle 44 entsprechend dem Gleichspannungsanteil des Empfänger-Ausgangssignales nachregelt, so dass Aenderungen der Strahlungsintensität der Strahlungsquelle 10 infolge Alterung, Temperaturschwankungen, Verstaubung oder Dejustierungen automatisch kompensiert werden können. Bei einer Ausbildung der Auswerteschaltung als Alarmschaltung kann so die Höhe der Alarmschwelle konstant gehalten werden. Von Besonderer Wichtigkeit kann eine solche Kompensation aber bei Ausbildung der Auswerteschaltung als Messschaltung sein, wenn der genaue Wert der Aerosol- oder Rauch-Dichte oder Konzentration zu messen ist.

Die Alarmschaltung 36 ist ausgebildet, im Alarmfall ein elektrisches Signal mit einer Frequenz im Hörbereich zu erzeugen, das der Treiberschaltung 42 des Luftbewegungsgenerators 4 zugeführt wird. Dadurch wird dieser veranlasst, zusätzlich zu den niederfrequenten Schwingungen unterhalb des Hörbereiches, die den periodischen Luftaustausch bewirken, ein akustisches Signal im Hörbereich abzustrahlen, das in der Nähe befindliche Personen auf den Alarmzustand aufmerksam macht. Dieses akustische Alarmsignal ist zweckmässigerweise so gestaltet, dass es möglichst grosse Aufmerksamkeit erregt, z.B. als Dissonanz, als Ton variabler Frequenz oder als Tonfolge. Stattdessen kann jedoch auch ein Sprachsignal durchgegeben werden, das in verständlicher Sprache auf den Gefahrenzustand aufmerksam macht und geeignete Verhaltensanweisungen verbreitet. Dazu kann die Alarmschaltung 36 einen entsprechenden Tonträger aufweisen.

Ausserdem kann vorgesehen sein, einen Alarmzustand von der Alarmschaltung einer Signalzentrale oder einem externen Alarmsignalgeber 39 zu signalisieren. Die Signalzentrale verarbeitet die von den einzelnen Detektoren eingehenden Signale und verteilt sie gegebenenfalls an entsprechende Stellen, so dass beispielsweise geeignete Bekämpfungsmassnahmen ergriffen werden können. Es kann auch vorteilhaft sein, die Alarmsignale anderer, z.B. benachbarter Detektoren 37 zugeleitet zu bekommen, so dass ein Detektor nicht nur den Zustand der eigenen Detektionsanordnung signalisiert, sondern auch den Zustand benachbarter Detektoren. Dabei ist es zweckmässig, wenn die akustischen Signale, die von den verschiedenen Detektoren ausgelöst werden, voneinander verschieden sind. Falls es sich um Sprachsignale handelt, kann durch eine geeignete Steuerung erreicht werden, dass der Ort des Gefahrenzustandes, d.h. der Ort des angesprochenen Detektors allgemeinverständlich bekanntgemacht wird.

Die Signale der einzelnen Alarmschaltungen 36, 37 können auch über eine Verarbeitungsschaltung 38 geleitet werden, die mit einem Mikroprozessor ausgerüstet sein kann, und die unter Berücksichtigung des Zustandes aller Detektoren das zweckmässigste Verhalten, z.B. die noch offenen Fluchtwege akustisch signalisiert.

: 269 a

Wie in der in Figur 2B gezeigten Variante der Schaltung nach Figur 2A kann das Wechsel-Ausgangssignal des Bandpassverstärkers 32 auch direkt dem Eingang eines Komparators 45 zugeführt werden, dessen Schwelle vom Driftkompensator 40 und der Referenzspannungsquelle 44 gesteuert wird. Die bei genügender Rauchdichte durch ein zur konstanten Triggerung des Komparators 45 ausreichendes Eingangssignal auftretende Impulsreihe wird dem Zähleingang eines Impulszählers 47 zugeführt. Im Normalfall, d.h. bei Abwesenheit eines Signales, wird der Impulszähler 47 kontinuierlich durch die Treiberschaltung 42 über eine Torschaltung 46 zurückgestellt. Bei Auftreten eines zur Triggerung des Komparators 45 genügend grossen Signales öffnet das Komparator-Ausgangssignal jedoch die Torschaltung 46 für eine durch ein RC-Zeitglied gegebene Zeitdauer. Der Impulszähler 47 zählt daher die Signal-Impulse und löst eine Alarmeinrichtung 48 aus, sobald ein vorbestimmter Zählerstand erreicht wird. Das Ausbleiben von Impulsen bewirkt, dass die Torschaltung 46 geschlossen und der Impulszähler 47 zurückgesetzt wird. Im Uebrigen ist die Funktion dieser Schaltungs-Variante analog zu derjenigen der vorher beschriebenen Schaltung nach Figur 2A.

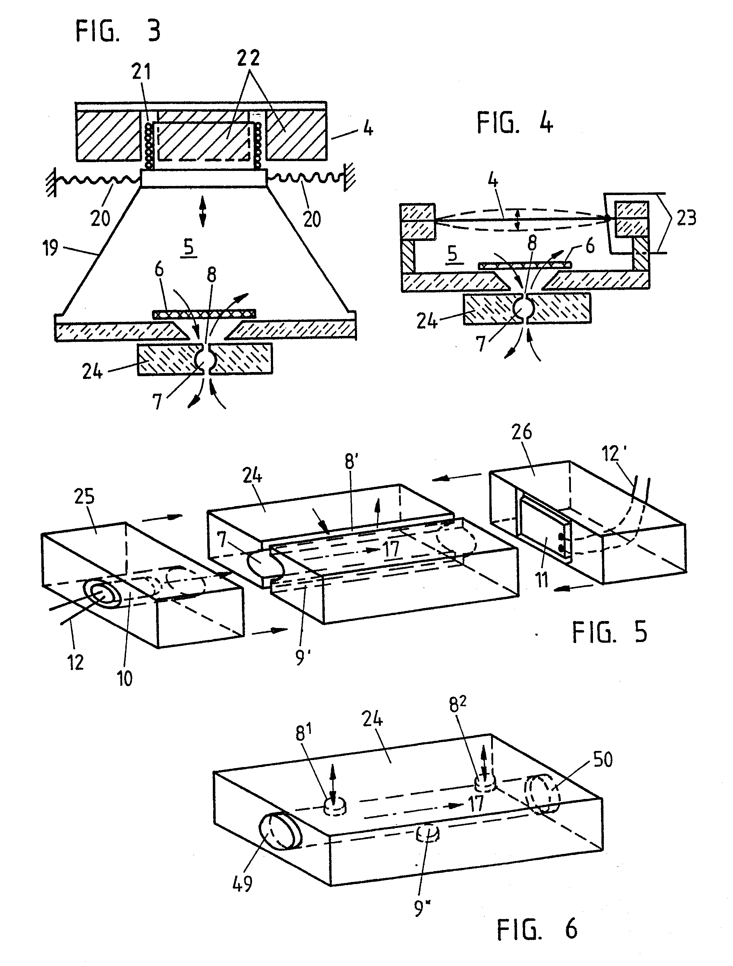

Figur 3 zeigt ein geeignetes Beispiel für den Aufbau einer Referenzkammer 5 mit zugehörigem Luftbewegungsgenerator 4. Eine konisch ausgebildete Wand 19 der Referenzkammer 5 ist an einer Spule 21 befestigt, die von einer federnden Halterung 20 getragen wird und in einen Ringspalt eines Permanent-Magneten 22 hineinragt. Bei Erregung der Spule 21 durch eine Wechselspannung gerät die Membran 19 in Schwingung, wie bei einem elektrodynamischen Lautsprecher, und ändert das Volumen der Referenzkammer 5 periodisch. Bei dieser Schwingung wird die überschüssige Luft aus der Referenzkammer 5 periodisch durch das Filter 6 und die Oeffnung 8 in die Messkammer 7 hineingedrückt und wieder zurückgesogen.

Figur 4 zeigt ein weiteres Beispiel einer Referenzkammer 5, wobei der Luftbewegungsgenerator 4 als bimorphe Piezo-Folie ausgebildet ist. Je nach der an den beiden Seiten mittels der Leitungen 23 angelegten Spannung erfährt diese Piezo-Folie eine entsprechende Auslenkung. Wird nun an die Leitungen 23 eine Wechselspannung angelegt, so gerät die Piezo-Folie in eine Schwingung mit einer Frequenz entsprechend der angelegten Wechselspannung und das Referenzkammer-Volumen schwankt mit der gleichen Frequenz.

In Figur 5 ist eine  zweckmässige  Ausbildung der Messkammer 7 dargestellt. Die Messkammer besteht in diesem Beispiel aus einer langgestreckten Bohrung in einem Kunststoffkörper 24. An die Längsseiten sind weitere Kunststoffkörper 25 und 26 angesetzt, die in zentralen Bohrungen die Strahlungsquelle 10 einerseits, und den Strahlungsempfänger 11 andererseits enthalten. Der Querschnitt der Bohrung 7 im Kunststoffkörper 24 entspricht dabei zweckmässigerweise der Form der Strahlungsquelle 10. Statt der im Beispiel gezeigten zylinderförmigen Bohrung kann aber auch ein anderer Querschnitt gewählt werden, z.B. ein quadratischer oder rechteckiger. Bei der beschriebenen Ausführung umfasst die Messkammer praktisch nur die eigentliche Transmissionsmessstrecke 17 und kein anderes Totvolumen.

An den flachen Seiten des Kunststoffkörpers 24 sind schlitzförmige, parallel zur Messkammer 7 oder Transmissionsmessstrecke 17 verlaufende Oeffnungen 8', 9' vorgesehen, mit denen die Messkammer 7 mit der Referenzkammer 5 einerseits, wie in den Figuren 3 und 4 angedeutet, in Verbindung steht, und andererseits mit dem Gehäuse-Inneren und der Aussenatmosphäre. Es zeigte sich, dass mit dieser besonderen Anordnung und Ausbildung der Oeffnungen ein besonders wirksamer und schneller Luftaustausch zwischen Messkammer und Referenzkammer erreicht werden kann, so dass es ausreichend ist, beide Volumina etwa gleich zu wählen. Selbst wenn das Volumen der Referenzkammer etwas kleiner gewählt wurde als das der Messkammer, war der Luftaustausch noch intensiv genug, um schon geringe Rauchkonzentrationen nachweisen zu können.

269 a

0131877

Zur Erzielung einer möglichst optimalen Wirkung ist es auch vorteilhaft, den zeitlichen Verlauf der Volumenänderung und des Luftwechsels an die gegebene Situation anzupassen. Für die beschriebene geometrische Anordnung hat es sich z.B. als zweckmässig erwiesen, einen dreieckförmigen Zeitverlauf zu wählen, jedoch erbrachten auch sinusförmige, rechteckförmige oder kompliziertere Zeitverläufe gute Resultate.

Wie in Figur 6 gezeigt, kann statt die Messkammer und die Referenzkammer durch einen Schlitz miteinander zu verbinden, in besonders vorteilhafter Weise zwischen den Kammern eine Reihe von Oeffnungen vorgesehen sein. Das Beispiel zeigt an der Referenzkammerseite entlang der Messstrecke 17 zwei Oeffnungen $8^1$, $8^2$ und gehäuseseitig eine zentrale Oeffnung 9". Stattdessen können auch entlang der Messstrecke 17 zwei gegeneinander versetzte Reihen von jeweils mehreren Oeffnungen vorgesehen sein.

Während zur Rauchdetektion oder zum Nachweis fester oder flüssiger Schwebeteilchen in einem Gas in der Regel polychromatische Strahlung mit einem breiten Spektrum ausreicht, ist zur Detektion einer bestimmten gasförmigen Komponente in einem Trägergas die Verwendung monochromatischer Strahlung im Strahlungsabsorptionsbereich der nachzuweisenden Komponente zweckmässig. Bei den Beispielen nach Figur 1 oder 5 kann dies z.B. dadurch erreicht werden, dass die Strahlungsquelle 10 als monochromatischer LASER ausgeführt wird, beim Beispiel nach Figur 6 dadurch dass auf der Seite der Strahlungsquelle oder des Empfängers, oder bei beiden, ein Filter 49, bzw. 50 mit geeignetem spektralen Durchlassbereich vorgesehen ist.

Es sei bemerkt, dass die Erfindung nicht auf die beschriebenen Beispiele der Rauch- oder Aerosoldetektion mit einer optischen Messstrecke als Aerosolsensor beschränkt ist, sondern mit analogen Vorteilen auch andere Aerosoldetektoren verwendet werden können, bei denen das Problem besteht, eine kleine Abweichung von einem grossen Normalwert zu bestimmen, z.B. eine Ionisationskammer, bei der sich der Ionenstrom in Abhängigkeit von der Aerosolkonzentration ändert.

Auch die Ausbildung des Sensors als rauch- oder gasempfindlicher Schwingquarz oder als kapazitiver Sensor ist im Rahmen des Erfindungsgedankens möglich, ebenso die Ausbildung als Streustrahlungs-Sensor, wobei die problemlose Trennung der modulierten, an Fremdpartikeln gestreuten Strahlung von gleichförmiger, nicht oder anders modulierter Störstrahlung, z.B. natürlichem Licht oder künstlichen Lichtquellen im Aussenbereich, von Vorteil ist.

Obwohl die Erfindung sich als besonders geeignet zur Rauch- und Aerosol-Detektion erwiesen hat, insbesondere zum Zwecke der Brandmeldung, so ist sie jedoch nicht darauf beschränkt. Auch andere Verwendungen der erfindungsgemässen Anordnung sind möglich, z.B. zur Detektion anderer Schwebeteilchen in einem Gas oder in Luft, etwa für die Kontrolle von Verbrennungsgasen oder der Luftverschmutzung. Auch zum Nachweis einer Fremdgaskomponente in einem Gas ist die Erfindung verwendbar, wenn der Sensor auf das nachzuweisende Fremdgas abgestimmt ist, und beispielsweise als selektiv auf das Fremdgas reagierender Gassensor ausgeführt ist, beispielsweise als Extinktions- oder Transmissionsstrecke mit monochromatischer Strahlung im Absorptionsbereich des Fremdgases, anstatt der zur Rauchdetektion üblichen polychromatischen Strahlung.

Auch der Luftbewegungsgenerator, sowie die elektrische Schaltung können im Rahmen des Erfindungsgedankens in abweichender Weise ausgebildet sein und zweckmässige und vorteilhafte Weiterbildungen umfassen, um die Störunanfälligkeit und die Betriebssicherheit zu verbessern. Mit den charakteristischen Merkmalen der Erfindung lässt sich so eine langzeitig erhalten bleibende Funktionssicherheit eines Fremdkomponenten-Detektors ohne die Notwendigkeit eines kontinuierlichen Ansaugverfahrens mit den erwähnten Nachteilen erreichen.

Patentansprüche

1. Anordnung zur Detektion einer in einem Gas enthaltenen Fremdkomponente mit einer für das zu untersuchende Gas zugänglichen Messkammer (7), die einen in Abhängigkeit vom Fremdkomponentengehalt ein Ausgangssignal abgebenden Sensor (17) aufweist, und einer das Ausgangssignal auswertenden elektrischen Schaltung (3), dadurch gekennzeichnet, dass eine für das zu untersuchende Gas nicht direkt zugängliche Referenzkammer (5) vorgesehen ist, die ein die zu detektierende Fremdkomponente nicht oder in geringerem Anteil aufweisendes Referenzgas enthält, die mit der Messkammer (7) mit wenigstens einer Oeffnung (8) in Verbindung steht, und die einen Luftbewegungsgenerator (4) aufweist, der periodisch abwechselnd Referenzgas aus der Referenzkammer (5) in die Messkammer (7) drückt, wobei das Gas in der Messkammer (5) zumindest teilweise durch Referenzgas ersetzt wird, und das Referenzgas wieder in die Referenzkammer (5) zurückzieht, wobei wiederum das zu untersuchende Gas in die Messkammer (7) eindringt, und dass die elektrische Schaltung (3) den Wechselanteil des Ausgangssignales des Sensors (17) als Kriterium für den Fremdkomponentengehalt auswertet.

2. Anordnung nach Anspruch 1, dadurch gekennzeichnet, dass der Luftbewegungsgenerator (4) eingerichtet ist, das Volumen der Referenzkammer (5) periodisch mit einer vorgegebenen Frequenz zu ändern.

3. Anordnung nach Anspruch 2, dadurch gekennzeichnet, dass ein Wandteil (4, 19) der Referenzkammer (5) schwingungsfähig ausgebildet ist, so dass bei einer Schwingung des Wandteiles (4, 19) eine periodische Volumenänderung der Referenzkammer (5) eintritt.

C 269 a

4. Anordnung nach Anspruch 3, dadurch gekennzeichnet, dass der schwingungsfähig ausgebildete Wandteil (4, 19) der Referenzkammer (5) als elektrodynamisch, elektrostatisch, piezoelektrisch oder thermomechanisch erregbare Membran ausgebildet ist.

5. Anordnung nach Anspruch 3 oder 4, dadurch gekennzeichnet, dass der schwingungsfähig ausgebildete Wandteil (4, 19) sowohl bei Frequenzen unterhalb des Hörbereiches als auch bei Frequenzen im Hörbereich schwingungsfähig ist.

6. Anordnung nach einem der Ansprüche 1 - 5, dadurch gekennzeichnet, dass das Volumen der Referenzkammer (5) mindestens gleich gross ist wie das Volumen der Messkammer (7).

7. Anordnung nach Anspruch 6, dadurch gekennzeichnet, dass das Verhältnis des Volumens der Messkammer (7) zum Volumen der Referenzkammer (5) mindestens 1 : 100 beträgt.

8. Anordnung nach einem der Ansprüche 1 - 7, dadurch gekennzeichnet, dass der Sensor als Transmissionsmessstrecke (17) mit einer elektromagnetische Strahlung aussendenden Strahlungsquelle (10) und einem deren Strahlung nach Durchquerung der Transmissionsmessstrecke (17) aufnehmenden Strahlungsempfänger (11) ausgeführt ist.

9. Anordnung nach Anspruch 8, dadurch gekennzeichnet, dass die Strahlungsquelle (10) elektromagnetische Strahlung in einem Spektralbereich auszusenden vermag, in welchem die nachzuweisende Fremdkomponente Strahlung absorbiert.

10. Anordnung nach Anspruch 9, dadurch gekennzeichnet, dass die Strahlungsquelle (10) ein monochromatische Strahlung aussendender LASER ist.

11. Anordnung nach Anspruch 8, dadurch gekennzeichnet, dass die Transmissionsmessstrecke (17) wenigstens ein Filter (49, 50) mit einem spektralen Durchlassbereich im Absorptionsbereich der nachzuweisenden Fremdkomponente aufweist.

12. Anordnung nach Anspruch 8, dadurch gekennzeichnet, dass die Messkammer (7) im Wesentlichen die Gestalt der Transmissionsmessstrecke (17) besitzt.

13. Anordnung nach Anspruch 12, dadurch gekennzeichnet, dass die die Messkammer (7) und die Referenzkammer (5) verbindende Oeffnung (8') als sich in Längsrichtung der Transmissionsmessstrecke (17) erstreckender Schlitz ausgebildet ist.

14. Anordnung nach Anspruch 13, dadurch gekennzeichnet, dass die Messkammer (7) eine weitere schlitzförmige, in Längsrichtung der Transmissionsmessstrecke (17) verlaufende Oeffnung (9') zur Verbindung mit der Aussenatmosphäre aufweist.

15. Anordnung nach Anspruch 12, dadurch gekennzeichnet, dass die Messkammer (7) und die Referenzkammer (5) in Längsrichtung der Transmissionsmessstrecke (17) durch eine Reihe von lochförmigen Oeffnungen ($8^1$, $8^2$) miteinander verbunden sind.

16. Anordnung nach einem der Ansprüche 1 - 15, dadurch gekennzeichnet, dass zwischen der Messkammer (7) und der Referenzkammer (5) ein für die zu detektierende Fremdkomponente undurchlässiges, für das Referenzgas jedoch durchlässiges Filter (6) vorgesehen ist.

17. Anordnung nach einem der Ansprüche 1 - 16, dadurch gekennzeichnet, dass die Referenzkammer ein Absorptionsmittel (18) für die zu detektierende Fremdkomponente aufweist.

18. Anordnung nach einem der Ansprüche 1 - 17, dadurch gekennzeichnet, dass die Auswerteschaltung eine frequenzselektive Schaltung (32) mit einem Durchlassbereich vorzugsweise bei der Frequenz der periodischen Volumenänderung der Referenzkammer (5) aufweist.

19. Anordnung nach Anspruch 18, dadurch gekennzeichnet, dass die Auswerteschaltung (3) eine Treiberschaltung (42) aufweist, die sowohl den Luftbewegungsgenerator (4) ansteuert, als auch den Durchlassbereich der frequenzselektiven Schaltung (32) beeinflusst.

20. Anordnung nach Anspruch 18 oder 19, dadurch gekennzeichnet, dass die elektrische Auswerteschaltung (3) einen Komparator (35) aufweist, der ein Ausgangs- oder Alarmsignal abgibt, wenn das mit einer vorgegebenen Zeitkonstante integrierte Ausgangssignal der frequenzselektiven Schaltung (32) einen vorbestimmten Schwellenwert übersteigt.

21. Anordnung nach Anspruch 20, dadurch gekennzeichnet, dass die elektrische Auswerteschaltung (3) eine vom Ausgangssignal des Sensors (17) angesteuerte Kompensationsschaltung (40, 44) aufweist, die den Schwellenwert für die Signalgabe entsprechend dem Gleichsignal des Sensors (17) nachregelt.

22. Anordnung nach Anspruch 18 oder 19, dadurch gekennzeichnet, dass die elektrische Auswerteschaltung (3) einen Komparator (45) aufweist, der eine Impulsreihe abgibt, wenn das Ausgangssignal der frequenzselektiven Schaltung (32) einen vorgegebenen Schwellenwert übersteigt, sowie einen Impulszähler (47), der die vom Komparator (45) abgegebenen Impulse zählt und bei Erreichen eines vorgegebenen Zählerstandes eine Alarmeinrichtung (48) auslöst.

23. Anordnung nach Anspruch 20 oder 22 , dadurch gekennzeichnet, dass die elektrische Auswerteschaltung (3) eine Alarmschaltung (36) aufweist, die bei Erhalt eines Ausgangssignales vom Komparator (35) sowohl einen Alarmsignalgeber (39) betätigt, als auch über eine Treiberschaltung (42) den Luftbewegungsgenerator zur Abgabe eines Signales im Hörbereich steuert.

169 a

24. Anordnung nach Anspruch 23, dadurch gekennzeichnet, dass das vom Luftbewegungsgenerator (4) bei Ansteuerung durch den Alarmsignalgeber (39) abgegebene Signal ein Sprachsignal ist.

25. Anordnung nach Anspruch 23 oder 24, dadurch gekennzeichnet, dass der Luftbewegungsgenerator (4), bzw. dessen Treiberschaltung (42) ausser von der zugeordneten Alarmschaltung (36) zusätzlich von Alarmschaltungen (37) anderer Detektions-Anordnungen ansteuerbar ist.

26. Anordnung nach Anspruch 25, dadurch gekennzeichnet, dass die Steuersignale der verschiedenen Alarmschaltungen (36, 37) sich in einer für die Herkunft von einer bestimmten Detektions-Anordnung charakteristischen Weise unterscheiden.

27. Anordnung nach Anspruch 25, dadurch gekennzeichnet, dass eine Verarbeitungsschaltung (38) vorgesehen ist, welche die Steuersignale der einzelnen Alarmschaltungen (36, 37) zu akustischen Verhaltensanweisungen verarbeitet, die dem Luftbewegungsgenerator (4), bzw. dessen Treiberschaltung (42) zugeleitet werden.

28. Verwendung der Anordnung nach einem der Ansprüche 1 - 27 zur Detektion der von einem Verbrennungsprozess erzeugten Rauchpartikel oder Brandaerosole in Luft.

29. Verwendung nach Anspruch 28, dadurch gekennzeichnet, dass bei Ueberschreitung eines vorbestimmten Schwellenwertes durch die Aerosolkonzentration oder Rauchdichte ein Brandalarmsignal ausgelöst wird.

30. Verwendung der Anordnung nach einem der Ansprüche 1 - 27 zur Detektion der Luftverschmutzung.

C 269 a

0131877

1/2

FIG. 1

FIG. 2A

FIG. 2B

FIG. 3

FIG. 4

FIG. 5

FIG. 6